# EUROPEAN PATENT APPLICATION

(11) **EP 4 763 966 A1**
(43) Date of publication of application: **24.06.2026**
(21) Application number: 26158762.0
(22) Date of filing: 16.02.2026
(51) Int. Cl.: C12M 1/00, C12M 1/34, G01N 33/04

(54) **A SYSTEM FOR AUTOMATED EVALUATION OF BIOLOGICAL CULTURES OF MILK SAMPLES**

(30) Priority: 17.12.2024 CZ 202442490 U
(71) Applicant: HERBAVITA CZ s.r.o., 37701 Jindrichuv Hradec (CZ)
(72) Inventor: Otahal, Ondrej, 37005 Ceske Budejovice (CZ); Jungwirth, Vaclav, 37701 Jindrichuv Hradec (CZ)
(74) Representative: Sedlák, Jirí

(57) **Abstract**

A system (1) for automated evaluation of biological cultures of milk samples (2) comprises an analyser (3) comprising a culture chamber (12), a tray (16) with openings (17) for placing cultured milk samples (2) in the culture chamber (12), a temperature control device (31), a humidity control device (32), a camera (14), and a frame (21) with a travel mechanism (22) carrying the camera (14). The system (1) further comprises a control unit (15) to which the temperature control device (31), the humidity control device (32), the travel mechanism (22), and the camera (14) are connected, further comprises at least one data storage (27) containing a database (5) of visual recordings of cultured milk samples (2) taken by the camera (14) and/or a software module (7) for evaluation of the visual recordings of cultured milk samples (2) and determination of their microbial pathogenicity using artificial intelligence.

## Description

### Field of the Invention

The present invention relates to the field of detection of microorganisms in foodstuffs, in particular to a system for automated evaluation of biological cultures of milk samples.

### Background of the Invention

Detection of the presence of undesirable microorganisms in the milk of dairy cows is essential for maintaining animal health, milk quality, and the safety of dairy products. If undesirable microorganisms are identified in milk, specific procedures are followed. These include diagnostics, treatment of animals, and preventive measures on the farm. Cultivation of the milk samples on Petri dishes is a common microbiological procedure used to detect and identify microorganisms that may be present in milk. This process is essential for quality control of dairy products, diagnosis of infections such as mastitis in dairy cows, detection of undesirable microorganisms such as Escherichia coli or Listeria, or for research purposes.

A milk sample is collected under sterile conditions, usually directly from the cow's udder or from a product line. It is important to ensure that there is no contamination of the sample. Petri dishes are filled with a specific culture medium that promotes bacterial growth, most commonly agar media. The milk is applied to the surface of the agar in the Petri dish using a sterile instrument. The dishes are incubated in a controlled environment (incubator) at a specific temperature, usually 35 to 37°C, depending on the type of microorganisms, for a period of 24 to 48 hours.

After incubation, the growth of microorganisms is examined, typically by visual inspection performed by an operator. Bacterial colonies can vary in appearance, such as colour, shape, or size, which provides clues for their identification. When the presence of undesirable microorganisms in dairy cow milk is detected, it is necessary to initiate treatment or adopt various preventive measures. If specific microorganisms are detected, they may be subjected to further tests, such as Gram staining, biochemical tests, or molecular tests. One option is the administration of broad-spectrum antibiotics to dairy cows; however, this approach has recently been abandoned, particularly due to the risk of developing antibiotic resistance. Therefore, identification of the presence of the specific strain of microorganism in milk is preferred. Testing aimed at identifying a specific microorganism in milk in order to initiate targeted treatment of dairy cows is time-consuming and expensive, as it is necessary to identify the specific strain of microorganism in order to apply targeted treatment. However, different types of microorganisms require different diagnostic methods, and identification of a specific microorganism may therefore take several days. This delays milk distribution and prolongs the treatment period of dairy cows. No sophisticated solution that would provide rapid and efficient milk diagnostics is currently known.

The object of the invention is therefore to provide a system for automated evaluation of biological cultures of milk samples that eliminates the above-mentioned drawbacks. The object of the invention is to provide a system capable of rapidly and efficiently evaluating the presence of undesirable microorganisms and simultaneously identifying the microorganisms using artificial intelligence. Furthermore, the object of the invention is to provide a system that enables simultaneous cultivation of microorganisms and identification within a single device. At the same time, the system should be universal so that it can be easily applied to all types of cultures and culture media.

### Summary of the Invention

The stated object is achieved by a system for automated evaluation of biological cultures of milk samples. The essence of the invention consists in that the system comprises an analyser for visual recording of cultured milk samples, which is configured as a lockable enclosure and comprises a culture chamber for accommodating milk samples, at least one tray with openings for placing cultured milk samples in the culture chamber, a device for controlling the temperature in the culture chamber, a device for controlling the humidity in the culture chamber, at least one camera, and a frame with a travel mechanism carrying the camera for selective visual recording of cultured milk samples placed on the tray. Furthermore, the system comprises at least one control unit to which the temperature control device, the humidity control device, the travel mechanism, and the camera are connected. The system further comprises at least one data storage containing a database of visual recordings of cultured milk samples taken by camera and/or a software module for evaluating visual recordings of cultured milk samples and determining their microbial pathogenicity using artificial intelligence.

The system of the present invention provides a comprehensive automated solution in which the process of milk sample cultivation, monitoring, and subsequent visual recording, such as shape, colour, sectors, evaluation of presence and type of pathogens, and pairing with additional data, such as data on the health status of dairy cows, data from performance monitoring, medication records, and other databases stored in cloud storage, is automated. Subsequently, the results are displayed to designated persons in a client section of a website or delivered in the form of a PDF to an email address or in the form of an SMS/notification to a mobile device. The customer simply places milk samples, i.e., a Petri dish with freshly spread milk, into the analyser and approximately after 24 hours can see whether any pathogen is present and, if applicable, which type is present on the dish and whether it is sensitive to antimicrobial treatments used on the farm. Furthermore, recommended procedures are provided to the customer, outlining steps that can be taken to promote the health of dairy cows, as well as systemic measures such as adjustment of the pressure in the milking parlour, improved housing systems, enhanced hygiene in the milking parlour, etc.

In a preferred embodiment, the control unit is part of the analyser.

In one preferred embodiment, the control unit is provided with a local user interface comprising at least some control or indication elements selected from the group consisting of buttons, an LCD display, a combination of buttons and an LCD display, LEDs, indicators, audio outputs, and voice control. The display shows data relating to temperature and humidity inside the analyser, as well as data relating to cultivation time, timing of visual recording, and whether the evaluation process of biological cultivation of milk samples is complete. The display further shows error and service messages, user settings such as Wi-Fi connection, etc., and the visual recording.

In another preferred embodiment, the control unit is connected to a web-based user interface for remote access. The web-based user interface allows users to view results of cultivations and identifications of pathogens present in milk, review previous visual recordings, read generated reports or comparisons, etc., using a computer, mobile phone, notebook, or tablet comprising a web-based user interface.

In one preferred embodiment, the data storage is part of the control unit.

In another preferred embodiment, the data storage is located in cloud storage, wherein the system further comprises a communication interface for transmitting visual recordings of cultured milk samples from the control unit of the analyser via a GSM network and/or LTE network and/or NB-IOT network and/or LPWAN network to the cloud storage, and a web-based user interface for remote access to the data storage. The web-based user interface allows users to view results of cultivations and identifications of pathogens present in milk, review previous visual recordings, read generated reports or comparisons, etc., on a user hardware device, such as a computer, mobile phone, notebook, or tablet comprising a communication interface for communication with the data storage. The system may further include an administrative hardware device selected from the group consisting of of a mobile phone, tablet, notebook, or computer comprising a communication interface for communication with the data storage and database.

In a preferred embodiment, the tray is configured as pull-out and removable from a lockable enclosure, wherein the frame of the lockable enclosure is provided with at least one pair of oppositely arranged profiles on which the pull-out and removable tray is slide-mounted. The profiles serve as supporting surfaces, so that by sliding the pull-out and removable tray onto these profiles, the pull-out and removable tray is positioned in the analyser for further processing.

In another preferred embodiment, the frame is provided on its sides with four pairs of oppositely arranged profiles placed one above the other, with one pull-out and removable tray arranged on each pair of oppositely arranged profiles. The analyser thus contains a total of four pull-out and removable trays placed one above the other. Each pull-out and removable carrier preferably comprises eight openings. The analyser therefore contains a total of 32 sampling sites where milk samples can be inserted for analysis in a single comprehensive device. This saves a significant amount of time and makes it possible to monitor a large number of milk samples, dairy cows, and their condition. Milk samples are applied to Petri dishes under sterile conditions. The openings in the pull-out and removable tray are smaller than the diameter of the Petri dish to prevent the Petri dishes from falling through the openings. A solidified culture medium, typically agar, is prepared in the Petri dishes, onto which the milk sample is applied. The Petri dish containing the milk sample is closed with a lid and turned upside down. This is done to prevent contamination of the milk sample by microorganisms that could be present on the lid of the Petri dish. The frames are easy to clean and easy to disassemble for cleaning, maintenance, and potential servicing.

The system preferably further comprises at least one light source for illuminating cultured milk samples in the culture chamber. In a preferred arrangement, the camera and the light source are integrated into a common photo module, which is mounted on a travel mechanism and is provided with two arms surrounding the tray from above and below. A light source is arranged in the upper arm and a camera for visual recording of milk samples is arranged in the lower arm. The photo module is preferably further provided with a device for identifying the occupancy of an opening in the tray and initiating the cultivation time of the milk samples, which may be any additional device such as a light beam or LED. However, this function may also be performed by the camera itself. The camera for visual recording of milk samples thus simultaneously serves as a device for identifying the occupancy of an opening in the tray and initiating the cultivation time of the milk samples, and further ensures detection of lens contamination to prevent loss or degradation of the milk sample, and in particular loss of time during diagnosis and treatment. The camera also serves as a camera for identification of the milk sample. Each milk sample is provided with an identification means, such as a QR code, which is paired with the identification number of the dairy cow. By means of the camera, the milk sample can be paired with the identification number of the dairy cow, which simplifies long-term monitoring of the condition of milk samples, as well as with data regarding previous conditions of milk samples from the given dairy cow or its previous treatment, etc. The system of the present invention enables printing of the identification means, which can be attached to the milk samples during sampling. The identification means may be located on the rim of the Petri dish or in the centre so that it is clearly visible and does not interfere with cultivation of the milk sample. Subsequently, the camera recognizes the given milk sample and pairs the visual recording with the identification number of the dairy cow.

The camera may be selected from the group consisting of a UV spectrum camera, a visible light camera, and a thermal imaging camera. The camera captures multiple visual recordings in the form of photographs or videos, such as different photographs or videos of the same milk sample, for example enlarged, under different lighting conditions, captured by different type of camera.

In a preferred embodiment, the enclosure is provided with a side shaft on one side of the frame. From this side, the frame is provided with passages into the side shaft for passage of the travel mechanism to individual trays located on different tiers of the frame. When making visual recording of milk samples located on different tiers of the frame, it is not necessary to pull the trays out of the frame, as passage of the travel mechanism to a higher tier is performed automatically.

The temperature control device and the humidity control device essentially serve to maintain prescribed conditions for successful cultivation of samples. These devices are preferably integrated into a heating element with a humidifier and a fan, which is arranged at the bottom of the culture chamber. The heating element generates heat as needed, which is essential for optimal cultivation conditions. At the same time, it includes an integrated humidifier that provides the necessary humidity in the analyser chamber, which is also essential for optimal cultivation conditions. The integrated fan ensures uniform distribution of heat and humidity throughout the culture chamber of the analyser, thereby creating optimal cultivation conditions for milk samples and any microorganisms present therein.

The system of the present invention combines, in a novel manner, visual information about the milk sample with any textual information, such as a report from a livestock specialist regarding the most recent condition of the farm or a specific animal, medication records, performance monitoring, veterinary report, feed analysis, or other laboratory records, as well as any additional metadata such as season of the year, changes in feeding, or health history of the cow, thereby enabling further improvement of detection, classification, or segmentation of pathogens.

The outputs of the system of the present invention thus include primary computer vision outputs such as classification or segmentation of undesirable microorganisms, i.e., pathogens. They further include extended outputs of significant pathogens based on their characteristics, such as severity, area, or development of colonies, and development in other dairy cows on the farm, which helps focus attention directly on the most important pathogens on the farm. They further include advanced outputs such as evaluation and further recommendations or predictions based on the outputs of the above-mentioned models in combination with a knowledge base regarding pathogens, dairy cows, best practices, feed sources, supplements, and medications available on the market.

The advantages of the system for automated evaluation of biological cultures of milk samples of the present invention lie in particular in its ability to rapidly and efficiently evaluate the presence of undesirable microorganisms and immediately identify them using artificial intelligence. In addition, it provides the possibility of simultaneous cultivation and identification of microorganisms within a single device. At the same time, the system should be universal so that it can be easily applied to all types of cultures and culture media.

### Explanation of drawings

The present invention will be explained in detail by means of the following figures where:
Fig. 1 shows a front view of the analyser,
Fig. 2 shows a rear view of the analyser,
Fig. 3 shows a view of the photo module,
Fig. 4 shows a view of the Petri dish,
Fig. 5 shows a view of the system of the present invention,
Fig. 6 shows a view of the system of the present invention.

### Example of the invention embodiments

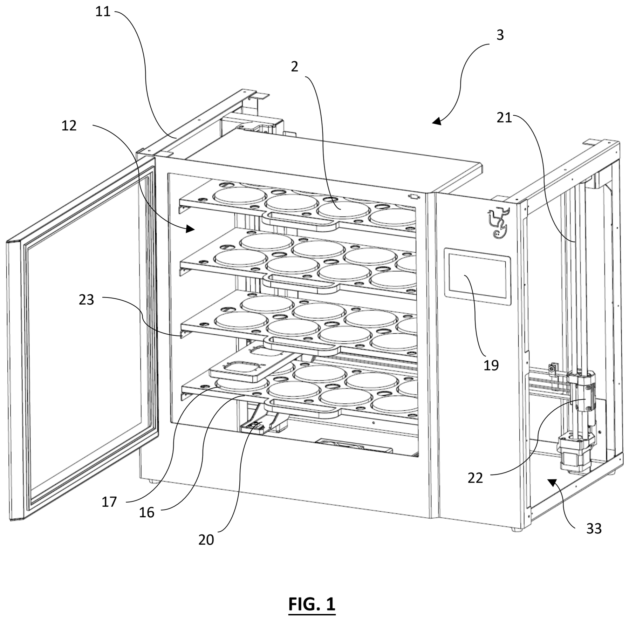

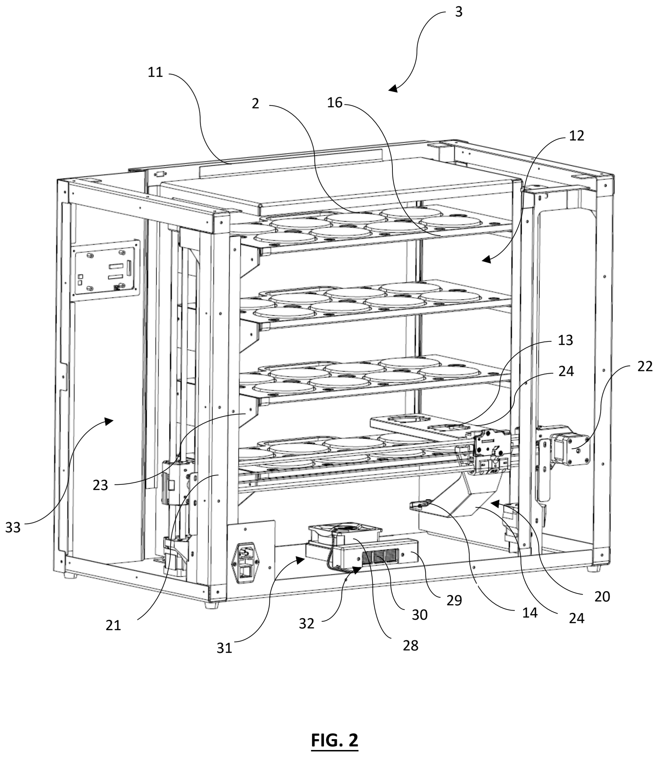

Fig. 1 and Fig. 2 show an analyser 3 for visual recording of cultured milk samples 2, which forms part of a system 1 for automated evaluation of biological cultures of milk samples 2 of the present invention. The analyser 3 is configured as a lockable enclosure 11, having an opening door on its front side and a touch LCD display 19 that displays temperature and humidity inside the analyser 3, cultivation time, error messages, visual recording check for lens contamination, and other information. In another embodiment, the control unit may further comprise buttons, LEDs, indicators, audio outputs, or voice control.

Inside the analyser 3 there is a culture chamber 12 containing a frame 21 into which pull-out and removable trays 16 are placed. The frame 21 is provided on its sides with four pairs of oppositely arranged profiles 23 attached one above the other, onto which four pull-out and removable trays 16 can be placed. Each tray 16 is designed as a flat rectangular plate with a handle on the longer side of the plate. Each tray 16 comprises eight openings 17 for placement of eight Petri dishes 18 containing milk samples 2 in two rows, i.e., each tray 16 has two rows of openings 17 with four openings 17 each. In total, there are thirty-two openings 17 in the analyser 3 for thirty-two Petri dishes 18 containing milk samples 2. In another embodiment, the analyser 3 may be designed with a different number of trays 16 having a different number of openings 17, i.e., the analyser 3 can cultivate and evaluate a different number of milk samples 2.

As shown in Fig. 2 and its rear view, a temperature control device 31 and a humidity control device 32 are located in the lower part of the culture chamber 12 of the analyser 3 and are formed in a single compact unit, namely a heating element 28 with an integrated humidifier 29 and a fan 30. The heating element 28 heats the culture chamber 12 of the analyser 3 to a temperature of 37°C, and the humidifier 29 maintains humidity inside the culture chamber 12 of the analyser 3 at 55%. Water is supplied to the humidifier 29 from a water tank (not shown). An integrated fan 30 distributes heat and water throughout the culture chamber 12 of the analyser 3. The cultivation of milk samples 2 takes 24 hours. In another embodiment, the cultivation time may be longer or shorter, depending on the pathogen in question.

A travel mechanism 22 is slide-mounted on the frame 21 in the analyser 3, on which the photo module 20 is arranged. The frame 21 is formed by a system of vertical and horizontal rods that are interconnected and constructed in such a way that the photo module 20 moves in the culture chamber 12 along the x and y axes, i.e., up or down and right or left.

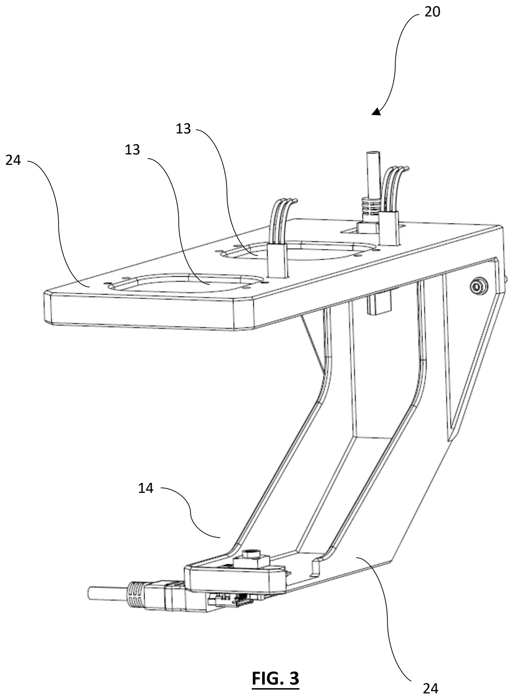

A detailed view of the photo module 20 is shown in Fig. 3. The photo module 20 comprises two arms 24 that surround the tray 16 from below and above. Two light sources 13, in this embodiment LEDs, arranged one behind the other are provided in the upper arm 24 of the photo module 20. Two light sources 13 are provided in the upper arm 24 of the photo module 20 because each tray 16 has two rows of openings 17, enabling illumination of two milk samples 2 at once in one position of the photo module 20. In the case of a different arrangement and number of openings 17 in the pull-out and removable tray 16, the number of light sources 13 in the upper arm 24 of the photo module 20 will also be adjusted. A camera 14 is mounted on the lower arm 24 of the photo module 20 for visual recording of milk samples 2, which in this embodiment is an RGB camera. The photo module 20 arranged in this way illuminates milk samples 2 located below using its upper arm 24 and light source 13 and takes photographs or videos of milk samples 2 located above using its lower arm 24 and camera 14, thereby creating an image or series of images of milk samples 2 that are subsequently sent to the data storage 27. In another embodiment, camera 14 for visual recording of milk samples 2 may also take a video of milk samples 2 that may be subsequently processed in the data storage 27 with a database 5 of visual recordings of cultured milk samples 2 taken by camera 14.

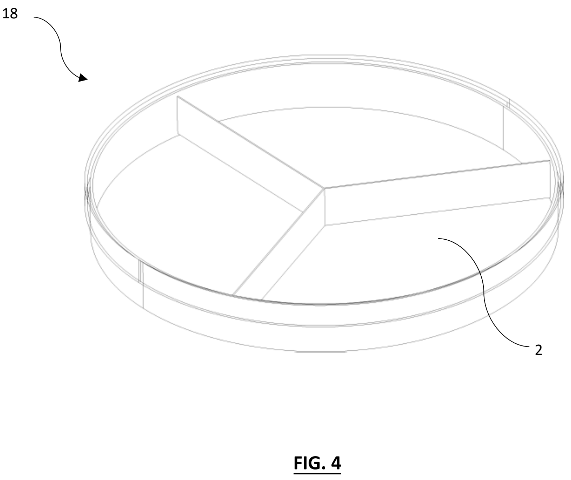

Fig. 4 shows a Petri dish 18 containing a milk sample 2, which is divided into three sectors. It is therefore possible to make a visual recording of up to three different cultured milk samples 2 in a single step.

### Example 1

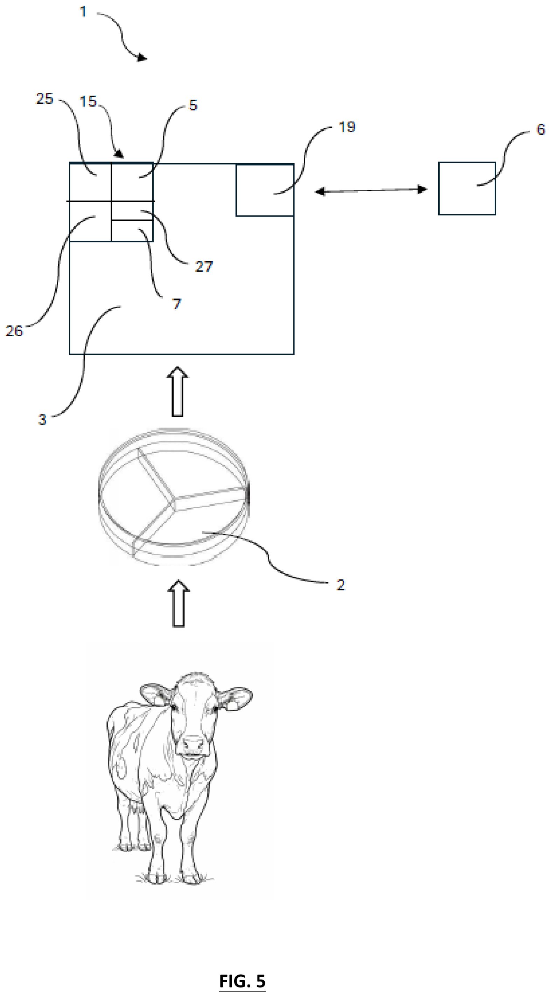

Fig. 5 shows a system 1 for automated evaluation of biological cultures of milk samples 2 of the present invention. As described above, milk samples 2 are placed in the analyser 3, where they are cultured and subsequently illuminated by a light source 13, and visually recorded by a camera 14 for making visual recording of milk samples 2, either in the form of a photograph, video, or a combination thereof. In this embodiment, the visual recordings of the cultured milk sample 2 are sent from camera 14 for visual recording of milk sample 2, to the data storage 27 in the control unit 15.

The control unit 15 comprises an integrated first microcomputer 25 connected to the travel mechanism 22 with the photo module 20 is connected for controlling movement of the photo module 20 in the culture chamber 12 along the x and y axes, as well as to the temperature control device 31 and the humidity control device 32. The control unit 15 further comprises an integrated second microcomputer 26 connected to the data storage 27 containing a database 5 of visual recordings of cultured milk samples 2 and a software module 7 for evaluation of the visual recordings of cultured milk samples 2 and determination of their microbial pathogenicity using artificial intelligence, which is also connected to the control unit 15. In another embodiment, only a database 5 of the visual recordings of cultured milk samples 2 taken by camera or only a software module 7 for evaluation of the visual recordings of cultured milk samples 2 and determination of their microbial pathogenicity using artificial intelligence is present on the data storage 27.

After visual recording of the cultured milk samples 2, these visual recordings are sent from the camera 14 for the visual recording of milk samples 2 to the control unit 15, where they are evaluated using artificial intelligence in the software module 7. The software module 7 receives the visual recording and sends it to its trained artificial intelligence. This trained artificial intelligence evaluates whether the visual recording is OK or whether any pathogen is present. If any undesirable pathogen is present, the user is informed via the display 19 of the analyser 3.

### Example 2

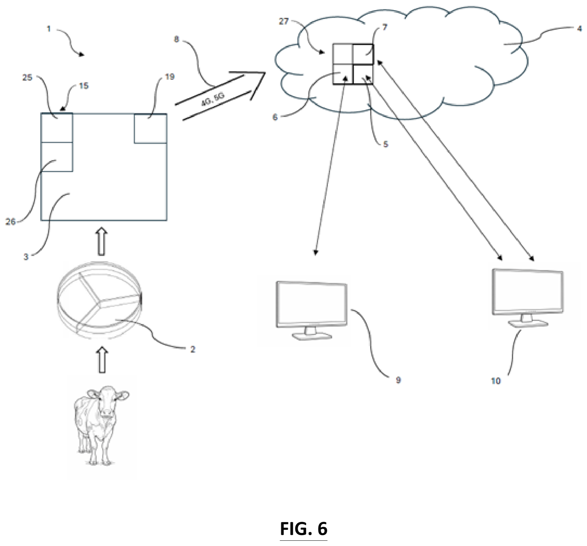

Fig. 6 shows a system 1 for automated evaluation of biological cultures of milk samples 2 of the present invention. As described above, milk samples 2 are placed in the analyser 3, where they are cultured and subsequently illuminated by a light source 13, and visually recorded by a camera 14 for making visual recording of milk samples 2, either in the form of a photograph, video, or a combination thereof.

In this embodiment, the visual recordings of the cultured milk sample 2 are sent from camera 14 for visual recording of milk sample 2 to the data storage 27 located in the cloud storage 4 via a communication interface 8 through a GSM network. In another embodiment, it is possible to use other networks, e.g., LTE, NB-IOT, or LPWAN. The cloud storage 4 comprises a web-based user interface 6 for remote access to the data storage 27, a database 5 of visual recordings of cultured milk samples 2, and a software module 7 for comparison of visual recordings of cultured milk samples 2 with sample visual recordings in database 5 and evaluation, and for communication with artificial intelligence. The software module 7 receives the visual recording and sends it to its trained artificial intelligence.

This trained artificial intelligence evaluates whether the visual recording is OK or whether any pathogen is present. If any undesirable pathogen is present, the user is informed on the user hardware device 9, in this case a computer, in the form of a PDF sent to an email address or, in another embodiment, a mobile phone or tablet in the form of an SMS/notification. In this embodiment, the administrative hardware device 10 is a computer on which encrypted and secured information regarding milk samples 2 and the condition of dairy cows is stored and can be evaluated at any time. In another embodiment, the administrative hardware device 10 may be a tablet or mobile phone.

### Industrial applicability

The system for automated evaluation of biological cultures of milk samples of the present invention can be used in particular by agricultural enterprises, veterinarians, livestock advisers, universities, and dairy processing facilities, as it is modular and portable. The system is suitable for routine office use and does not expose users to a hazardous environment.

### List of index reference numerals

1 system
2 milk sample
3 analyser
4 cloud storage
5 database
6 web-based user interface
7 software module
8 communication interface
9 user hardware device
10 administrative hardware device
11 enclosure
12 culture chamber
13 light source
14 camera
15 control unit
16 tray
17 opening
18 Petri dish
19 display
20 photo module
21 frame
22 travel mechanism
23 profile
24 arm
25 first microcomputer
26 second microcomputer
27 data storage
28 heating element
29 humidifier
30 fan
31 temperature control device
32 humidity control device
33 shaft

## Claims

1. A system (1) for automated evaluation of biological cultures of milk samples (2), **characterized in that** it comprises an analyser (3) for visual recording of cultured milk samples (2), which is configured as a lockable enclosure (11) and comprises a culture chamber (12) for accommodating milk samples (2), at least one tray (16) with openings (17) for placing cultured milk samples (2) in the culture chamber (12), a temperature control device (31) for controlling temperature in the culture chamber (12), a humidity control device (32) for controlling humidity in the culture chamber (12), at least one camera (14), and a frame (21) with a travel mechanism (22) carrying the camera (14) for selective visual recording of cultured milk samples (2) in the tray (16), wherein the system (1) further comprises at least one control unit (15) to which the temperature control device (31), the humidity control device (32), the travel mechanism (22), and the camera (14) are connected, and further comprises at least one data storage (27) containing a database (5) of visual recordings of cultured milk samples (2) taken by the camera (14) and/or a software module (7) for evaluation of the visual recordings of cultured milk samples (2) and determination of their microbial pathogenicity using artificial intelligence.

2. The system (1) of claim 1, **characterized in that** the control unit (15) is part of the analyser (3).

3. The system (1) of claim 1 or 2, **characterized in that** the control unit (15) is provided with a local user interface comprising at least some control or indication elements selected from the group consisting of buttons, an LCD display (19), a combination of buttons and an LCD display (19), LEDs, indicators, audio outputs, and voice control.

4. The system (1) of claim 1 or 2, **characterized in that** the control unit (15) is connected to a web-based user interface (6) for remote access.

5. The system (1) of any of claims 1 to 4, **characterized in that** the data storage (27) is part of the control unit (15).

6. The system (1) of any of claims 1 to 4, **characterized in that** the data storage (27) is located on a cloud storage (4), wherein the system (1) further comprises a communication interface (8) for transmitting visual recordings of cultured milk samples (2) from the control unit (15) of the analyser (3) via a GSM network and/or LTE network and/or NB-IOT network and/or LPWAN network to the cloud storage (4), and a web-based user interface (6) for remote access to the data storage (27).

7. The system (1) of any of claims 1 to 6, **characterized in that** the tray (16) is configured as pull-out and removable from a lockable enclosure (11), wherein the frame (21) of the lockable enclosure (11) is provided with at least one pair of oppositely arranged profiles (23) on which the pull-out and removable tray (16) is slide-mounted.

8. The system (1) of claim 7, **characterized in that** the frame (21) is provided on its sides with four pairs of oppositely arranged profiles (23) placed one above the other, with one pull-out and removable tray (16) arranged on each pair of oppositely arranged profiles (23).

9. The system (1) of claim 7 or 8, **characterized in that** each pull-out and removable tray (16) comprises eight openings (17) for accommodating cultured milk samples (2) in the culture chamber (12).

10. The system (1) of any of claims 1 to 9, **characterized in that** it further comprises at least one light source (13) for illuminating cultured milk samples (2) in the culture chamber (12).

11. The system (1) of claim 10, **characterized in that** the camera (14) and the light source (13) are integrated into a common photo module (20), which is mounted on the travel mechanism (22) and is provided with two arms (24) surrounding the tray (16) from its upper side and lower side, wherein the light source (13) is arranged in the upper arm (24) and the camera (14) for visual recording of cultured milk samples (2) is arranged in the lower arm (24).

12. The system (1) of claim 11, **characterized in that** the photo module (20) is further provided with a device for identifying the occupancy of an opening (17) in the pull-out and removable tray (16) and for initiating the cultivation time of the milk samples (2).

13. The system (1) of any of claims 1 to 12, **characterized in that** the enclosure (11) is provided on one side of the frame (21) with a side shaft (33), wherein the frame (21) is provided on this side with passages into the side shaft (33) for passage of the travel mechanism (22) to individual trays (16) located on different tiers of the frame (21).

14. The system (1) of any of claims 1 to 13, **characterized in that** the temperature control device (31) and the humidity control device (32) are integrated in a heating element (28) with a humidifier (29) and a fan (30), which is mounted at the bottom of the culture chamber (12).
